# EUROPEAN PATENT SPECIFICATION

(11) **EP 2 890 457 B1**
(45) Date of publication and mention of the grant of the patent: **19.01.2022**
(21) Application number: 13832673.1
(22) Date of filing: 29.08.2013
(51) Int. Cl.: A61N 5/10

(54) **BRACHYTHERAPY APPLICATOR DEVICE**
BRACHYTHERAPIEAPPLIKATOR
DISPOSITIF D'APPLICATEUR DE CURIETHÉRAPIE

(30) Priority: 29.08.2012 US 201261694758 P
(43) Date of publication of application: 08.07.2015
(73) Proprietor: Best Medical International, Inc., Springfield, VA 22153 (US)
(72) Inventor: FILLMORE, Spencer J., Greenacres, WA 99016 (US); LEE, Christopher M., Spokane Valley, WA 99037 (US)
(74) Representative: DenK iP bv
(86) International application number: PCT/US2013/057413
(87) International publication number: WO 2014/036337

(56) References cited:
- WO-A2-2011/011731
- FR-A1- 2 401 669
- US-A- 4 292 960
- US-A- 4 331 131
- US-A- 5 012 357
- US-A1- 2008 064 916
- US-A1- 2008 064 916
- US-A1- 2012 123 188

## Description

### BACKGROUND OF THE INVENTION

### 1. Field of the Invention

The present invention relates generally to an applicator device for use in delivering radiation therapy, and more particularly to a brachytherapy applicator device for use in supplying a radiation source to an internal tumor.

### 2. Description of the Related Art

Brachytherapy is a form of cancer treatment in which radiation sources are placed inside a patient's body to irradiate an internal tumor. Thus, in brachytherapy, a radioactive source is placed in or around a tumor. Brachytherapy thus has the advantage of delivering high doses of ionizing radiation to small volumes of tissue, combined with a rapid fall off of dose such that distant tissue is spared. It thus has provided excellent results for localized control of various cancers, including, for example, cancer of the vagina, cervix, or uterus.

Afterloading is a commonly used radiation delivery technique wherein a nonradioactive applicator is first positioned in the treatment site and then the applicator is loaded with a radiation source. Once the applicator is correctly positioned in the patient the applicator is connected to an afterloader machine, which contains the radioactive source, and the radioactive source is provided to the applicator through a series of connecting guide tubes. The radioactive source remains in place for a specified length of time and then is drawn back through the tubes to the afterloader machine. The applicator is then removed from the treatment site. US2008/0064916 A1 describes methods and devices for providing the delivery of radioactive therapeutics to a cervix using a split ring applicator.

Careful placement of the applicator, and thus, placement of the radioactive source is important to allow for localized and precise irradiation of the tumor. Additionally, ease of placement and positioning of the applicator is essential to improve the comfort of the patient during brachytherapy treatment.

### SUMMARY OF THE INVENTION

The invention is as defined in the appended claims. An applicator device is provided. In one example, the applicator device includes a connecting unit. The connecting unit includes a first connector coupled to a first colpostat and a second connector coupled to a second colpostat. The first connector includes a first contact portion and a body portion, the first contact portion being immovably fixed to the body portion. The second connector includes a second contact portion and a third contact portion, the second contact portion being configured to contact and pivotally engage the first contact portion. The first connector further includes a fourth contact portion configured to contact the third contact portion. The second contact portion and the third contact portion are disposed between the first contact portion and the fourth contact portion, and the first contact portion are disposed in closer proximity to a point of delivery of a radiation source than the second, third, or fourth contact portions.

Example embodiments are described in more detail below with reference to the appended figures.

### BRIEF DESCRIPTION OF THE DRAWINGS

FIG. 1 illustrates a Fletcher-type brachytherapy applicator device for use in intracavity application of a radioactive source according to a first embodiment.
FIG. 2 illustrates a Henschke-type brachytherapy applicator device for use in intracavity application of a radioactive source according to a second embodiment.
FIG. 3 illustrates a bottom perspective view of a first connector.
FIG. 4 illustrates a side elevation view of the first connector.
FIG. 5 illustrates a top plan view of the first connector.
FIG. 6 illustrates a side elevation view of the first connector.
FIG. 7 illustrates a bottom perspective view of a first connector.
FIG. 8 illustrates a bottom plan view of first connector.
FIG. 9 illustrates a bottom perspective view of a second connector.
FIG. 10 illustrates a bottom plan view of the second connector.
FIG. 11 illustrates a top plan view of the second connector.
FIG. 12 illustrates a side elevation view of the second connector.
FIG. 13 illustrates a fastening unit.
FIG. 14. illustrates a spacing bracket tandem clamp.
FIGS. 15A, 15B, and 15C illustrate another embodiment of the applicator device.
FIGS. 16A, 16B, and 16C illustrate another embodiment of the applicator device.
FIGS. 17A, 17B, and 17C illustrate another embodiment of the applicator device.
FIGS. 18A, 158, and 18C illustrate another embodiment of the applicator device.
FIGS. 19A and 19B illustrate another embodiment of the applicator device.
FIGS. 20A and 20B illustrate another embodiment of the applicator device.

### DETAILED DESCRIPTION OF THE INVENTION

In the detailed description, like reference characters denote like parts in the several figures.

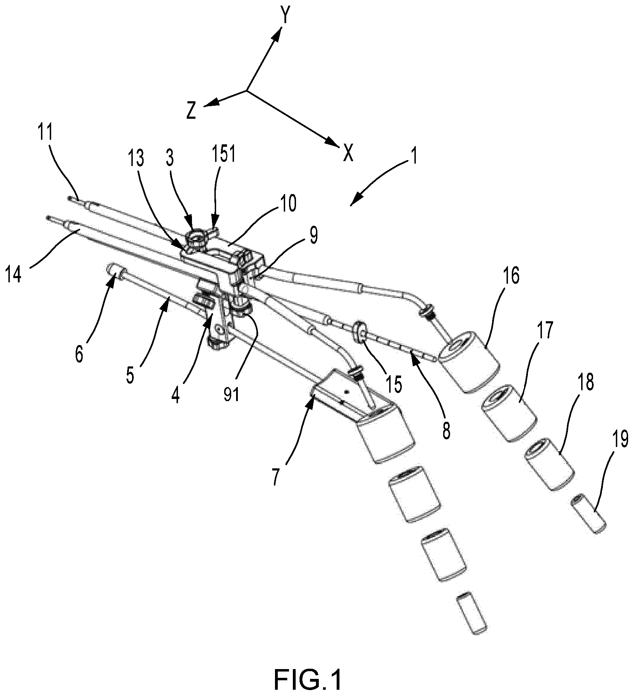

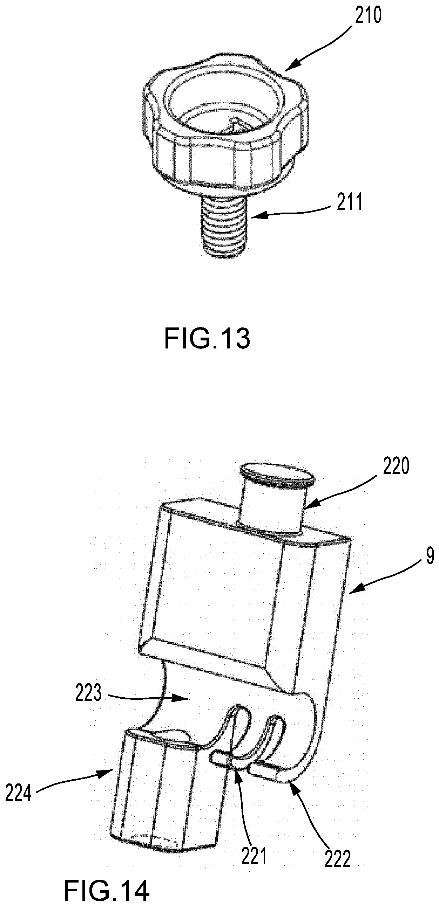

FIG. 1 illustrates a Fletcher-type brachytherapy applicator device 1 for use in intracavity application of a radioactive source according to a first embodiment. Applicator device 1 comprises a connecting unit including a left colpostat spacing bracket, hereinafter referred to as a first connector 10, coupled to left colpostat 11, and a right colpostat spacing bracket, hereinafter referred to as a second connector 13, coupled to right colpostat 14. Colpostats 11 and 14, which may be spread laterally, provide for intravaginal positioning of tandem 8, through which the radioactive source is provided to the cervix. Spacing bracket tandem clamp 9, also shown in FIG. 14, couples central tandem 8 to left colpostat spacing bracket 10. And cervical stop 15 is attached toward a distal end of central tandem 8.

The applicator device further includes a rectal paddle 7 coupled to a rectal paddle bracket block 4 by a rectal paddle shaft 5. A rectal paddle end knob 6 is fixed to a proximal end of rectal paddle shaft 5. And rectal paddle bracket block 4 is coupled to central tandem 8.

When colpostats 11 and 14 are properly positioned, fastening unit 3 may be fastened, which binds arm member 151 of second connector 13 to a top surface of first connector, to fix the distal ends of colpostats 11 and 14 in position along the vaginal wall.

As shown in FIG. 1, cylindrical ovoids 16, 17, 18, 19 of various sizes may be attached to the distal ends of left colpostat 11 and right colpostat 14, and the distal ends of left colpostat 11 and right colpostat 14 are angled. In the first embodiment, central tandem 8 is straight at the distal end. However, in other embodiments, the distal end of central tandem 8 may be curved.

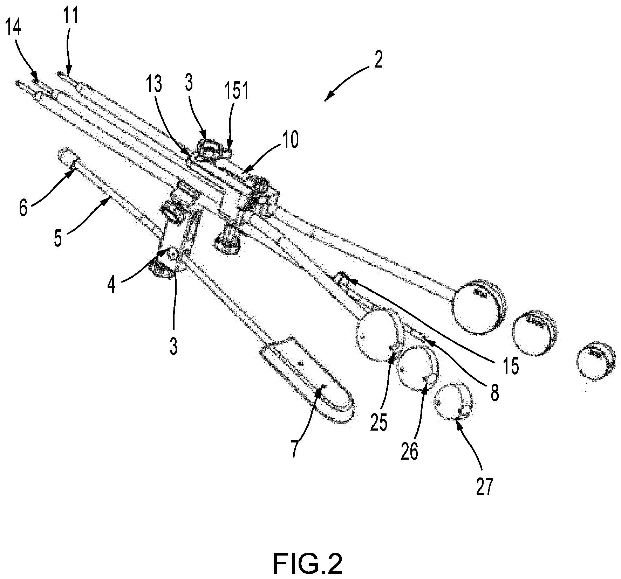

FIG. 2 illustrates a Henschke-type brachytherapy applicator device 2 for use in intracavity application of a radioactive source according to another embodiment. Like reference characters of FIG. 2 show the same or similar features as FIG. 1. But applicator device 2 includes semi-spherical ovoids 25, 26, 27 of various size that may be attached to the distal ends of left colpostat 11 and right colpostat 14. In the second embodiment, central tandem 8 is straight at the distal end. However, in other embodiments, the distal end of central tandem 8 may be curved.

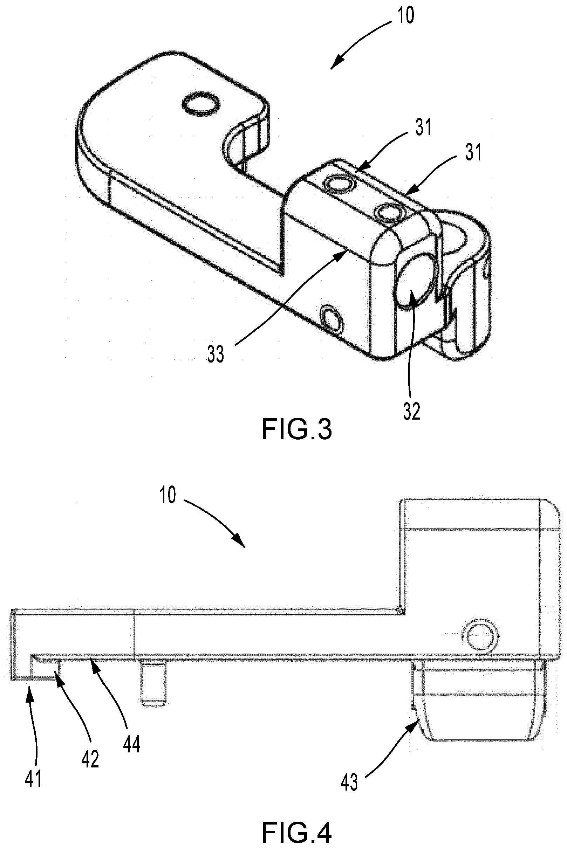

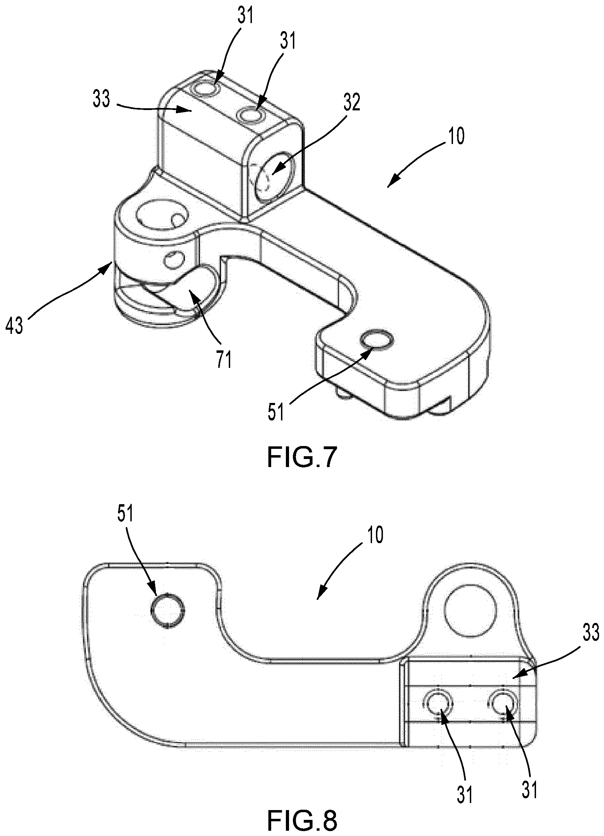

FIGS. 3 and 7 illustrates bottom perspective views of left colpostat spacing bracket or first connector 10. FIG. 8 shows a bottom plan view of first connector 10. Left colpostat 11 is attached to connector 10 by left colpostat coupling unit 33. Left colpostat 11 is placed in opening 32 and fasteners (not shown) may be used to fasten left colpostat 11 to left colpostat coupling unit 33 through through-holes 31.

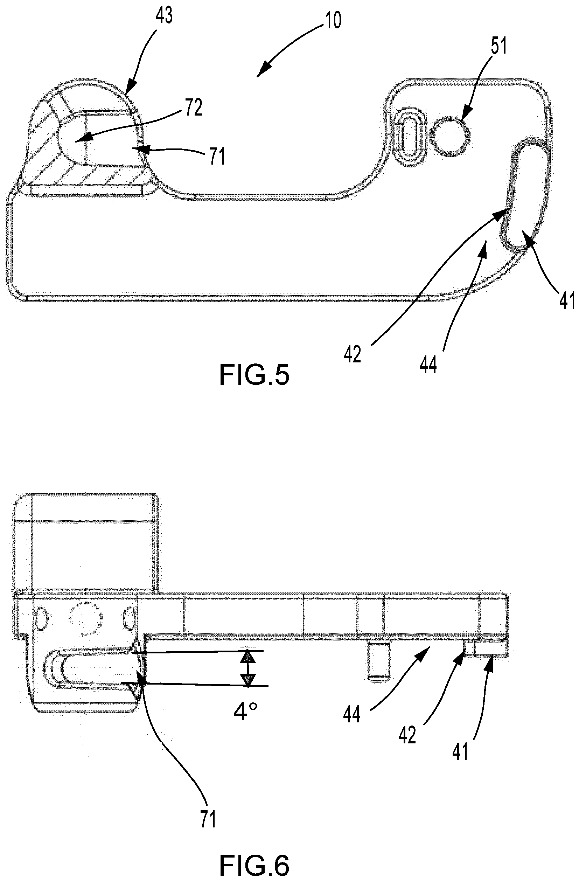

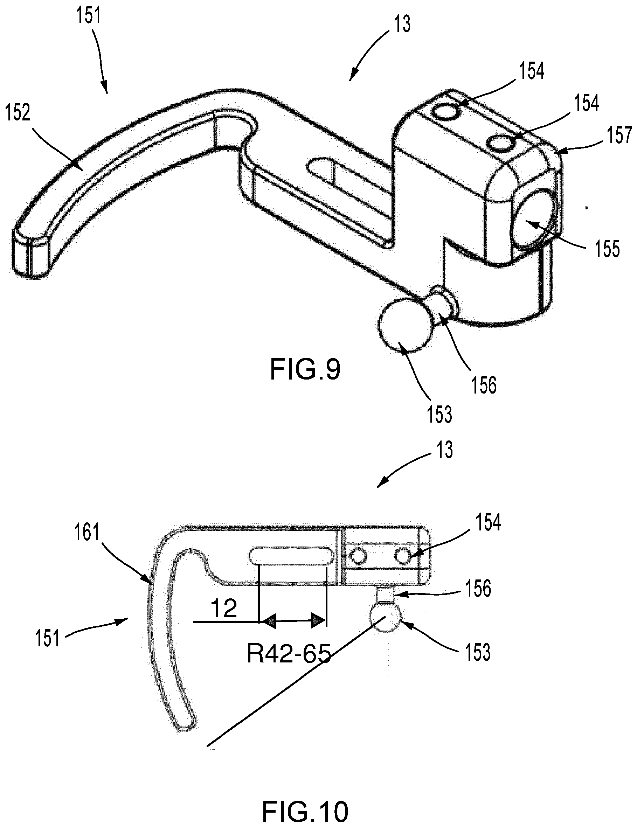

FIGS. 4 and 6 show respective side elevational views of connector 10 showing raised portion 41, which protrudes from a top surface 44 of connector 10. Raised portion 41 has a lateral surface 42. FIGS. 5 and 6 further show concavity 71, which in this embodiment, is a socket member including concave surface 72, configured to engage a convex member, in this embodiment, the convex member being ball member 153 protruding from second connector 13 (as shown in FIGS. 9-12). Concavity 71 opens toward the proximal end of connector 10 and toward raised portion 41, which is positioned toward the proximal end of first connector 10.

FIG. 5 shows a top plan view of connector 10 showing raised portion 41, which protrudes from top surface 44 of connector 10. Raised portion 41 has lateral surface 42. FIG. 5 further shows hole 51, which is configured to receive a shaft 211 of fastening unit 3, as shown in FIG. 21. In this embodiment, shaft 211 and hole 51 are respectively threaded.

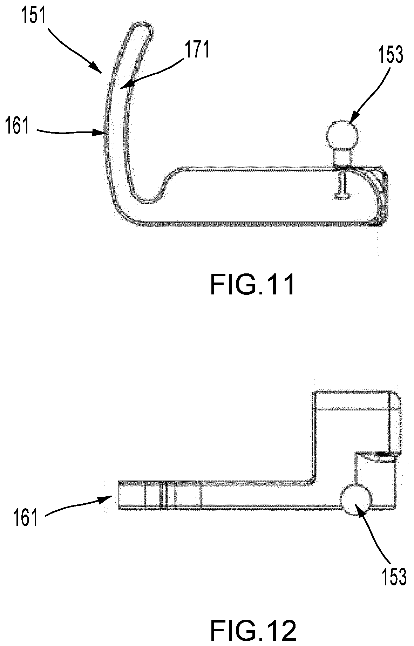

FIG. 9 illustrates a bottom perspective view of right colpostat spacing bracket or second connector 13. Second connector 13 includes a protruding arm member 151 having a bottom surface 152, which is configured to come in contact with top surface 44 of first connector 10. Additionally, as shown in FIGS. 10, 11, and 12, arm member 151 has proximal lateral surface 161, which is configured to contact lateral surface 42 of raised portion 41 on the first connector 10. Fastening unit 3 is provided to fasten second connector 13 to first connector 10 at an appropriate angle after a desired angle between left colpostat 11 and right colpostat 14 is achieved by adjustment. As shown in FIG. 21, fastening unit 51 may include a threaded bolt having threaded shaft 211 and head 210, the underside of the head 210 (not shown) coming into contact with the top surface 171 of arm member 151, FIG. 11 showing top surface 171 of arm member 151 in a top plan view of second connector 13.

Bottom surface 152 of arm member 151 of second connector 13 being in contact with top surface 44 of first connector 10 during adjustment and while fastened, and proximal lateral surface 161 protruding arch member 151 right colpostat spacing bracket 13 being in contact with lateral surface 42 of raised portion 41 on left colpostat spacing bracket 10 during adjustment and while fastened, lateral surface 42 preventing travel of the right colpostat spacing bracket 13 in the proximal direction during adjustment and after being fastened.

As shown in FIG. 9, right colpostat 14 is coupled to second connector 13 by right colpostat coupling unit 157. Right colpostat 14 is placed in opening 155 and fasteners (not shown) may be used to fasten right colpostat 14 to right colpostat coupling unit 157 through through holes 154.

Further, ball member 153 is coupled to second connector 13 by stem 156. And ball member 153 is configured to be received by concave member 43, operatively coupling first connector 10 to second connector 13, as shown in FIGS. 1 and 2.

As shown in FIG. 14, spacing bracket tandem clamp 9 includes attachment head 220, configured to be coupled to either first connector 10 or second connector 13. Spacing bracket tandem clamp further includes center finger 221, configured to couple within the tandem receiving portion 223.

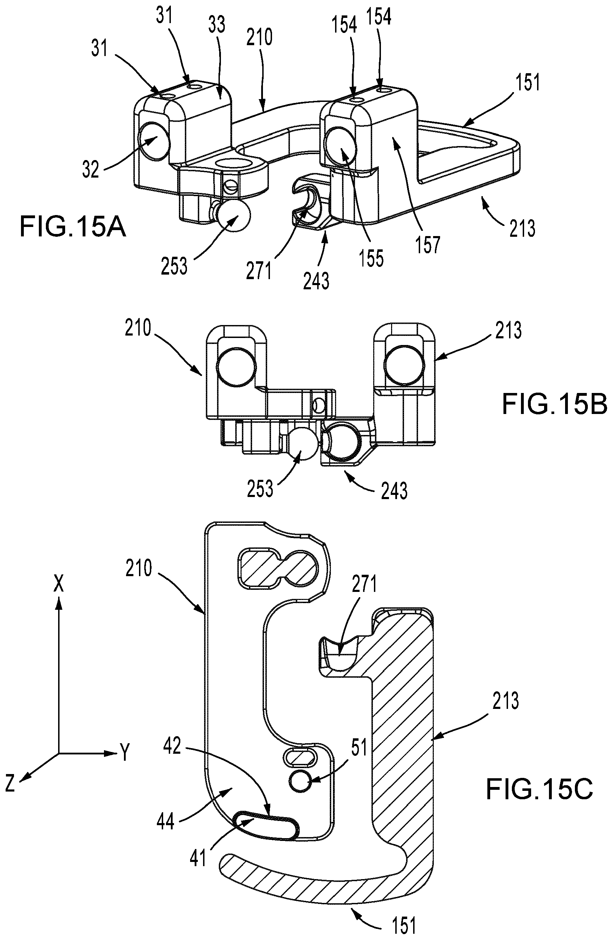

In another embodiment, as shown in FIGS. 15A, 15B, and 15C, first connector 210 includes a convex member, in this embodiment ball member 253, and second connector 213 includes concavity 243 including concave surface 271. In this embodiment, concavity 243 is a socket. Like reference characters of other embodiments denote like or similar parts in FIGS. 15A, 15B, and 15C.

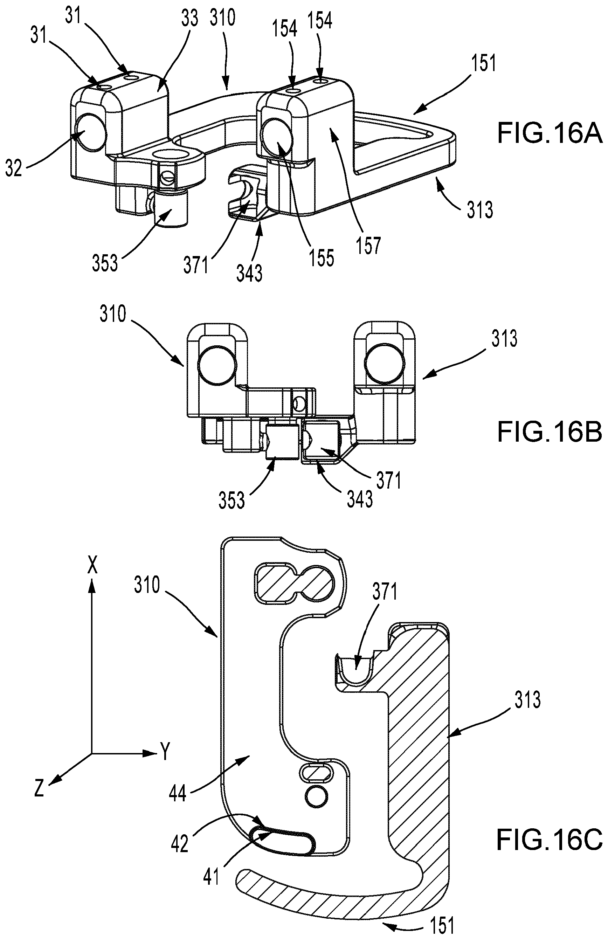

In another embodiment, as shown in FIGS. 16A, 16B, and 16C, first connector 310 includes a convex member, in this embodiment cylindrical shaft member 353, and second connector 313 includes concavity 343 including concave surface 371. In this embodiment, concavity 343 is a housing member configured to engage cylindrical shaft member 353. cylindrical shaft member 353 is perpendicular or nearly perpendicular to the first surface 44 of first connector 310. Like reference characters of other embodiments denote like or similar parts in FIGS. 16A, 16B, and 16C.

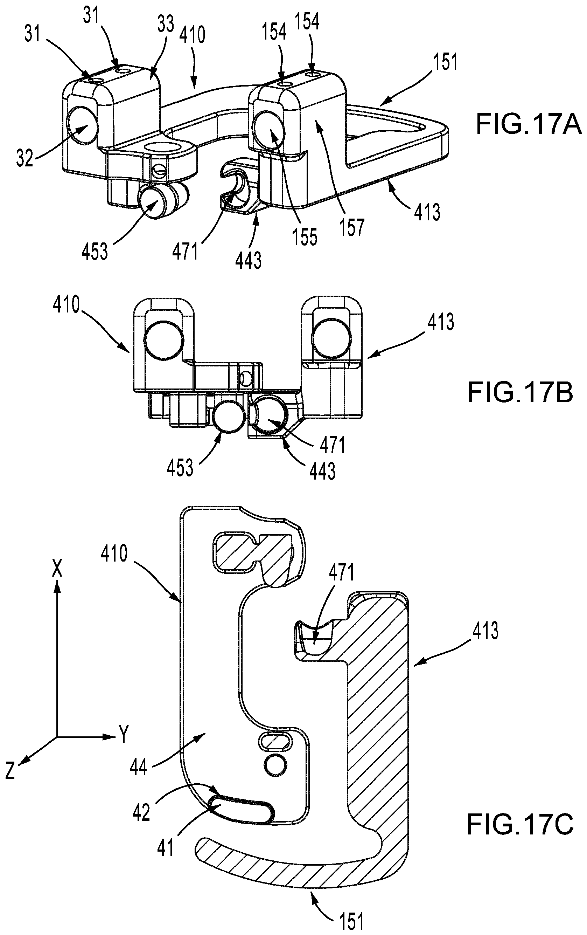

In another embodiment, as shown in FIGS. 17A, 17B, and 17C, first connector 410 includes a convex member, in this embodiment cone member 453, and second connector 413 includes concavity 443 including a concave surface 471. In this embodiment, concavity 443 is a conical housing member configured to engage cone member 453. Like reference characters of other embodiments denote like or similar parts in FIGS. 17A, 17B, and 17C.

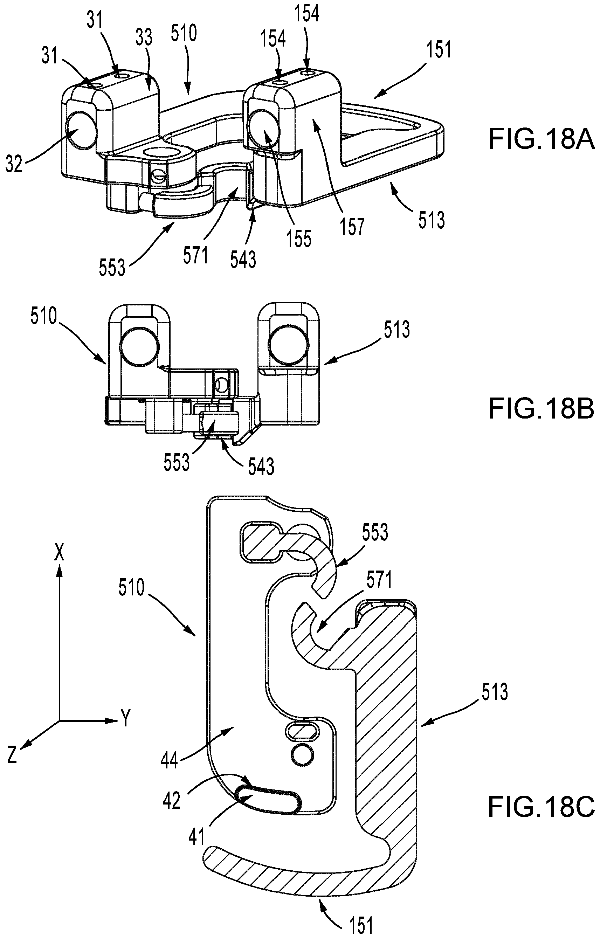

In another embodiment, as shown in FIGS. 18A, 18B, and 18C, first connector 510 includes a convex member, in this embodiment protruding hook member 553, and second connector 513 includes concavity 543 including concave surface 571. In this embodiment, concavity 543 is a receiving hooking member configured to engage protruding hook member 553. Like reference characters of other embodiments denote like or similar parts in FIGS. 18A, 18B, and 18C.

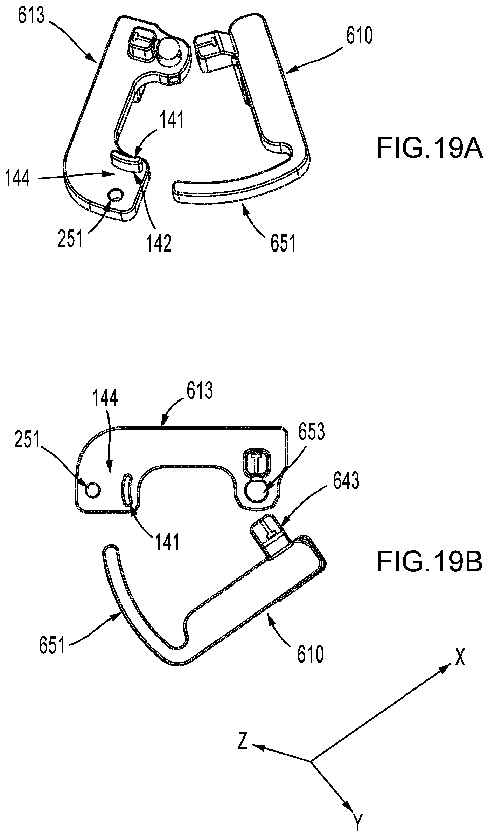

In another embodiment, as shown in FIGS. 19A and 19B, first connector 610 includes arm member 651, which is configured to contact lateral surface 142 of raised portion 141, protruding from first surface 144 of second connector 613. Additionally, through hole 251 is formed toward a proximal end portion of second connector 613. Like reference characters of other embodiments denote like or similar parts in FIGS. 19A and 19B.

In another embodiment, as shown in FIGS. 19A and 19B, first connector 610 includes arm member 651, which is configured to contact lateral surface 142 of raised portion 141, protruding from first surface 144 of second connector 613. Additionally, through hole 251 is formed toward a proximal end portion of second connector 613, through hole 215 being configured to receive a fastening unit 210. Like reference characters of other embodiments denote like or similar parts in FIGS. 19A and 19B.

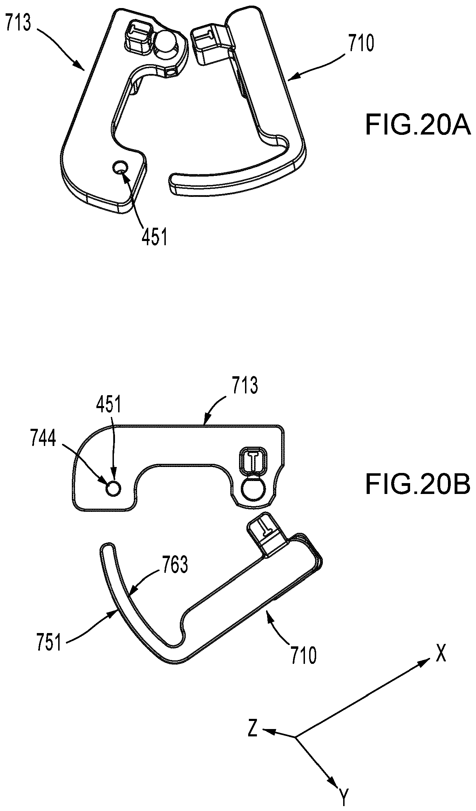

In another embodiment, as shown in FIGS. 20A and 20B, first connector 710 includes arm member 751 having a distal lateral surface 763. Distal lateral surface 763 is perpendicular or nearly perpendicular to first surface 744 of second connector 713. Thus, arm member 751 is configured to contact shaft 211 of fastening unit 210 (both not shown), through hole 451 being configured to receive fastening unit 210. contact lateral surface 142 of raised portion 141, protruding from first surface 144 of second connector 613. Additionally, through hole 251 is formed toward a proximal end portion of second connector 613, through hole 215 being configured to receive a fastening unit 210. Like reference characters of other embodiments denote like or similar parts in FIGS. 19A and 19B.

In the above embodiments, the first connector includes a first contact portion and a body portion, the first contact portion being immovably fixed to the body portion. The first contact portion may include either a concave member or a convex member. And the second connector includes a second contact portion, the second contact portion being configured to contact and pivotally engage the first contact portion, the second contact portion being either a concave member if the first contact portion includes a convex member or a convex member if the first contact portion includes a concave member.

The second connector further includes a third contact portion, and the first connector further includes a fourth contact portion, the fourth contact portion being configured to contact the third contact portion.

In the above embodiments, the second contact portion and the third contact portion are disposed between the first contact portion and the fourth contact portion, the first contact portion being disposed in closer proximity to a point of delivery of a radiation source than the second, third, or fourth contact portions.

In some embodiments, the first contact portion includes a concave member, for example a socket, a housing member configured to engage a cylindrical shaft member, or receiving hooking member. But instead of a concave member, the first contact portion may include a convex member, for example, a ball, a cylindrical shaft member, or a protruding hooking member.

And in some embodiments, the third contact portion includes an arm member, while the fourth contact portion includes the raised portion protruding from the first surface of the first connector. However, in other embodiments, such as that shown in FIGS. 19A and 19B, the third contact portion includes a raised portion protruding from the first surface of the second connector.

Additionally, in other embodiments, the third contact portion includes an arm member and the fourth contact portion includes the shaft of the fastening unit, or, as shown in FIGS. 20A and 20B, in another embodiment, the third contact portion includes the shaft of the fastening unit and the fourth contact portion includes an arm member having a distal surface configured to contact the shaft of the fastening unit.

The arrangement of the first connector including a first contact portion and a fourth contact portion and the second connector including a second contact portion and a third contact portion, and the second contact portion and the third contact portion being disposed between the first contact portion and the fourth contact portion, allows the first connector to operably couple to the second connector in such a way as to prevent travel in the X-direction, as shown in the Cartesian coordinate of FIGS. 1, 15C, 16C, 17C, 18C, 19B, and 20B. However, while engaged, the first connector and the second connector may pivot in the Y-direction, as shown in the Cartesian coordinate of FIGS. 1, 15C, 16C, 17C, 18C, 19B, and 20B. Additionally, embodiments incorporating the ball and socket, as either the first contact portion or the second contact portion, or vice-versa, allow freedom of movement in both the Y-direction and the Z-direction, while the first colpostat and the second colpostat are being positioned along the vaginal wall. In any case, positioning and placement of the applicator may be performed with more ease, precision, and efficiency due to the first contact portion being immovably coupled to the first connector or right colpostat spacing bracket.

While various embodiments of the present invention have been described above, and although various examples and experiments disclosing various aspects of the present invention have been disclosed, it should be understood that they have been presented by way of example only, and not limitation. It will be understood by those skilled in the art that various changes in form and details may be made therein without departing from the scope of the invention as defined in the appended claims. The embodiments, aspects and examples which do not fall within the scope of the claims are provided for illustrative purpose only and do not form part of the present invention. The invention is defined in the claims as follows.

## Claims

1. A brachytherapy applicator device (1) comprising:
a radiation source configured to be disposed to a point of delivery ,
a connecting unit including a first connector (10) coupled to a first colpostat (11) and a second connector (13) coupled to a second colpostat (14), wherein
the first connector (10) includes a first contact portion (71) and a body portion, the first contact portion (71) being immovably fixed to the body portion,
the second connector includes a second contact portion (153) and a third contact portion (151), the second contact portion (153) being configured to contact and pivotally engage the first contact portion (71),
the first connector (10) further includes a fourth contact portion (41) configured to contact the third contact portion (151), and
the second contact portion (153) and the third contact portion (151) are disposed between the first contact portion and the fourth contact portion, the first contact portion (71) being disposed closer to the point of delivery of the radiation source than the second, third, or
fourth contact portions are disposed to the point of delivery of the radiation source,
**characterized in that** the first contact portion (71) pivots around the second contact portion (153) and wherein the first contact portion (71) includes a socket member, and the second contact portion (153) includes a ball member.

2. The brachytherapy applicator device according to claim 1, wherein the first contact portion includes a concave portion configured to engage a convex portion of the second contact portion.

3. The brachytherapy applicator device according to claim 1, wherein the second contact portion is immovably fixed to a body portion of the second connector.

4. The brachytherapy applicator device according to claim 1, wherein the third contact portion includes an arm member, the arm member including a first surface configured to contact a first surface of the first connector and a second surface configured to contact the fourth contact portion or wherein the fourth contact portion includes an arm member, the arm member including a first surface configured to contact a first surface of the second connector and a second surface configured to contact the third contact portion.

5. The brachytherapy applicator device according to claim 1, wherein the third contact portion includes an arm member, the arm member including a first surface configured to contact a first surface of the first connector and a second surface configured to contact the fourth contact portion, and
wherein the fourth contact portion includes a raised portion protruding from the first connector, the raised portion including a lateral surface configured to engage the second surface of the arm member, or
wherein the fourth contact portion includes a fastening unit configured to fasten the arm member to the first connector, the fastening unit including a shaft configured to contact the second surface of the arm member,
wherein the first surface of the arm member is nearly perpendicular to the second surface of the arm member, and the first surface of the arm member is nearly parallel to the first surface of the first connector.

6. The brachytherapy applicator device according to claim 1, wherein the fourth contact portion includes an arm member, the arm member including a first surface configured to contact a first surface of the second connector and a second surface configured to contact the third contact portion, and
wherein the third contact portion includes a raised portion protruding from the second connector, the raised portion including a lateral surface configured to engage the second surface of the arm member, or
wherein the third contact portion includes a fastening unit configured to fasten the arm member to the second connector, the fastening unit including a shaft configured to contact the second surface of the arm member, wherein the first surface of the arm member is nearly perpendicular to the second surface of the arm member, and the first surface of the arm member is nearly parallel to the first surface of the second connector.

7. The brachytherapy applicator device according to claim 1, wherein the third contact portion includes an arm member, the arm member including a first surface configured to contact a first surface of the first connector and a second surface configured to contact the fourth contact portion, and wherein the fourth contact portion includes a raised portion protruding from the first connector, the raised portion including a lateral surface configured to engage the second surface of the arm member, the applicator device further comprising a fastening unit configured to fasten the arm member to the first connector, the fastening unit including a shaft arranged on a side of the arm member opposite from the raised portion, the arm member configured to operatively pass between the shaft of the fastening unit and the lateral surface of the raised portion.

8. The brachytherapy applicator device according to claim 1, wherein the fourth contact portion includes and arm member, the arm member including a first surface configured to contact a first surface of the second connector and a second surface configured to contact the third contact portion, and wherein the third contact portion includes a raised portion protruding from the second connector, the raised portion including a lateral surface configured to engage the second surface of the arm member, the applicator device further comprising a fastening unit configured to fasten the arm member to the second connector, the fastening unit including a shaft arranged on a side of the arm member opposite from the raised portion, the arm member configured to operatively pass between the shaft of the fastening unit and the lateral surface of the raised portion.

9. The brachytherapy applicator device according to claim 7, wherein the fastening unit is a threaded bolt, an underside surface of a head of the threaded bolt being configured to contact a top surface of the arm member and bind the arm member to the first surface of the first connector or to the first surface of the second connector.

10. The brachytherapy applicator device according to claim 7, wherein the fourth contact portion includes a fastening unit configured to fasten the arm member to the first connector, the fastening unit including a shaft configured to contact the second surface of the arm member, wherein the fastening unit is a threaded bolt, an underside surface of a head of the threaded bolt being configured to contact a top surface of the arm member and bind the arm member to the first surface of the first connector, or
wherein the third contact portion includes a fastening unit configured to fasten the arm member to the second connector, the fastening unit including a shat configured to contact the second surface of the member, wherein the fastening unit is a threaded bolt, an underside surface of a head of the threaded bolt being configured to contact a top surface of the arm member and bind the arm member to the first surface of the second connector.

11. The brachytherapy applicator device according to any of the previous claims,
wherein a distal end of the first colpostat and a distal end of the second colpostat are configured to be spread laterally by movement operation of the first connector or the second connector, or
wherein the applicator device furthermore comprises a first ovoid coupled to a distal end of the first colpostat, and a second ovoid coupled to a distal end of the second colpostat or
wherein the applicator device furthermore comprises a paddle support unit coupled to the connecting unit, the paddle support unit including a paddle coupled to a paddle shaft, or wherein the applicator device comprises a first ovoid coupled to a distal end of the first colpostat, and a second ovoid coupled to a distal end of the second colpostat and wherein the first ovoid and the second ovoid are each cylindrical or semi-cylindrical, or wherein the first ovoid and the second ovoid are each spherical or semi-spherical or
wherein the applicator device furthermore comprises a tandem coupled to the connecting unit or a tandem coupled to the connection unit and to the first connector or the second connector.

## Patentansprüche

1. Eine Brachytherapie-Applikatorvorrichtung (1), umfassend:
eine Strahlungsquelle, die so konfiguriert ist, dass sie an einem Abgabepunkt angeordnet werden kann,
eine Verbindungseinheit einschließlich einem ersten Verbinder (10), der mit einem ersten Kolpostat (11) gekoppelt ist, und einem zweiten Verbinder (13), der mit einem zweiten Kolpostat (14) gekoppelt ist, wobei
der erste Verbinder (10) einen ersten Kontaktabschnitt (71) und einen Körperabschnitt einschließt, wobei der erste Kontaktabschnitt (71) unbeweglich an dem Körperabschnitt befestigt ist,
der zweite Verbinder einen zweiten Kontaktabschnitt (153) und einen dritten Kontaktabschnitt (151) einschließt, wobei der zweite Kontaktabschnitt (153) so konfiguriert ist, dass er den ersten Kontaktabschnitt (71) kontaktiert und schwenkbar in Eingriff nimmt,
der erste Verbinder (10) weiter einen vierten Kontaktabschnitt (41) einschließt, der so konfiguriert ist, dass er den dritten Kontaktabschnitt (151) kontaktiert, und
der zweite Kontaktabschnitt (153) und der dritte Kontaktabschnitt (151) zwischen dem ersten Kontaktabschnitt und dem vierten Kontaktabschnitt angeordnet sind, wobei der erste Kontaktabschnitt (71) näher an dem Abgabepunkt der Strahlungsquelle angeordnet ist als der zweite, dritte oder
vierte Kontaktabschnitt an dem Abgabepunkt der Strahlungsquelle angeordnet sind,
**dadurch gekennzeichnet, dass** der erste Kontaktabschnitt (71) um den zweiten Kontaktabschnitt (153) schwenkt und wobei der erste Kontaktabschnitt (71) ein Buchsenelement und der zweite Kontaktabschnitt (153) ein Kugelelement einschließt.

2. Die Brachytherapie-Applikatorvorrichtung nach Anspruch 1, wobei der erste Kontaktabschnitt einen konkaven einschließt aufweist, der so konfiguriert ist, dass er mit einem konvexen Abschnitt des zweiten Kontaktabschnitts in Eingriff kommt.

3. Die Brachytherapie-Applikatorvorrichtung nach Anspruch 1, wobei der zweite Kontaktabschnitt unbeweglich an einem Körperabschnitt des zweiten Verbinders befestigt ist.

4. Brachytherapie-Applikatorvorrichtung nach Anspruch 1, wobei der dritte Kontaktabschnitt ein Armelement einschließt, wobei das Armelement eine erste Oberfläche einschließt, die so konfiguriert ist, dass sie eine erste Oberfläche des ersten Verbinders kontaktiert, und eine zweite Oberfläche, die so konfiguriert ist, dass sie den vierten Kontaktabschnitt kontaktiert, oder wobei der vierte Kontaktabschnitt ein Armelement einschließt, wobei das Armelement eine erste Oberfläche einschließt, die so konfiguriert ist, dass sie eine erste Oberfläche des zweiten Verbinders kontaktiert, und eine zweite Oberfläche, die so konfiguriert ist, dass sie den dritten Kontaktabschnitt kontaktiert.

5. Die Brachytherapie-Applikatorvorrichtung nach Anspruch 1, wobei der dritte Kontaktabschnitt ein Armelement einschließt, wobei das Armelement eine erste Oberfläche, die so konfiguriert ist, dass sie eine erste Oberfläche des ersten Verbinders kontaktiert, und eine zweite Oberfläche einschließt, die so konfiguriert ist, dass sie den vierten Kontaktabschnitt kontaktiert, und
wobei der vierte Kontaktabschnitt einen erhabenen Abschnitt einschließt, der von dem ersten Verbinder vorsteht, wobei der erhabene Abschnitt eine seitliche Oberfläche einschließt, die so konfiguriert ist, dass sie mit der zweiten Oberfläche des Armelements in Eingriff kommt, oder
wobei der vierte Kontaktabschnitt eine Befestigungseinheit einschließt, die so konfiguriert ist, dass sie das Armelement an dem ersten Verbinder befestigt, wobei die Befestigungseinheit einen Schaft einschließt, der so konfiguriert ist, dass er die zweite Oberfläche des Armelements kontaktiert,
wobei die erste Oberfläche des Armelements nahezu senkrecht zu der zweiten Oberfläche
des Armelements ist und die erste Oberfläche des Armelements nahezu parallel zu der ersten Oberfläche des ersten Verbinders ist.

6. Die Brachytherapie-Applikatorvorrichtung nach Anspruch 1, wobei der vierte Kontaktabschnitt ein Armelement einschließt, wobei das Armelement eine erste Oberfläche, die so konfiguriert ist, dass sie eine erste Oberfläche des zweiten Verbinders kontaktiert, und eine zweite Oberfläche einschließt, die so konfiguriert ist, dass sie den dritten Kontaktabschnitt kontaktiert, und
wobei der dritte Kontaktabschnitt einen erhabenen Abschnitt einschließt, der von dem zweiten Verbinder vorsteht, wobei der erhabene Abschnitt eine seitliche Oberfläche einschließt, die so konfiguriert ist, dass sie mit der zweiten Oberfläche des Armelements in Eingriff kommt, oder
wobei der dritte Kontaktabschnitt eine Befestigungseinheit einschließt, die so konfiguriert ist, dass sie das Armelement an dem zweiten Verbinder befestigt, wobei die Befestigungseinheit einen Schaft einschließt, der so konfiguriert ist, dass er die zweite Oberfläche des Armelements kontaktiert, wobei die erste Oberfläche des Armelements nahezu senkrecht zu der zweiten Oberfläche des Armelements ist, und die erste Oberfläche des Armelements nahezu parallel zu der ersten Oberfläche des zweiten Verbinders ist.

7. Die Brachytherapie-Applikatorvorrichtung nach Anspruch 1, wobei der dritte Kontaktabschnitt ein Armelement einschließt, wobei das Armelement eine erste Oberfläche, die so konfiguriert ist, dass sie eine erste Oberfläche des ersten Verbinders kontaktiert, und eine zweite Oberfläche, die so konfiguriert ist, dass sie den vierten Kontaktabschnitt kontaktiert, einschließt, und wobei der vierte Kontaktabschnitt einen erhöhten Abschnitt einschließt, der von dem ersten Verbinder vorsteht, der erhabene Abschnitt eine seitliche Oberfläche einschließt, die so konfiguriert ist, dass sie mit der zweiten Oberfläche des Armelements in Eingriff kommt, wobei die Applikatorvorrichtung weiter eine Befestigungseinheit umfasst, die so konfiguriert ist, dass sie das Armelement an dem ersten Verbinder befestigt, wobei die Befestigungseinheit einen Schaft einschließt, der auf einer Seite des Armelements gegenüber dem erhabenen Abschnitt eingerichtet ist, wobei das Armelement so konfiguriert ist, dass es betriebsmäßig zwischen dem Schaft der Befestigungseinheit und der seitlichen Oberfläche des erhabenen Abschnitts hindurchgeht.

8. Die Brachytherapie-Applikatorvorrichtung nach Anspruch 1, wobei der vierte Kontaktabschnitt ein Armelement einschließt, wobei das Armelement eine erste Oberfläche, die so konfiguriert ist,
dass sie eine erste Oberfläche des zweiten Verbinders kontaktiert, und eine zweite Oberfläche, die so konfiguriert ist, dass sie den dritten Kontaktabschnitt kontaktiert, einschließt, und wobei der dritte Kontaktabschnitt einen erhabenen Abschnitt einschließt, der von dem zweiten Verbinder vorsteht, der erhabene Abschnitt eine seitliche Oberfläche einschließt, die so konfiguriert ist, dass sie mit der zweiten Oberfläche des Armelements in Eingriff kommt, wobei die Applikatorvorrichtung weiter eine
Befestigungseinheit umfasst, die so konfiguriert ist, dass sie das Armelement an dem zweiten Verbinder befestigt, wobei die Befestigungseinheit einen Schaft einschließt, der auf einer Seite des Armelements gegenüber dem erhabenen Abschnitt eingerichtet ist, wobei das Armelement so konfiguriert ist, dass es betriebsmäßig zwischen dem Schaft der Befestigungseinheit und der seitlichen Oberfläche des erhabenen Abschnitts hindurchgeht.

9. Die Brachytherapie-Applikatorvorrichtung Anspruch 7, wobei die Befestigungseinheit ein Gewindebolzen ist, wobei eine untere Oberfläche eines Kopfes des Gewindebolzens so konfiguriert ist, dass sie eine obere Oberfläche des Armelements kontaktiert und das Armelement mit der ersten Fläche des ersten Verbinders oder mit der ersten Oberfläche des zweiten Verbinders verbindet.

10. Die Brachytherapie-Applikatorvorrichtung nach Anspruch 7, wobei der vierte Kontaktabschnitt eine Befestigungseinheit einschließt, die so konfiguriert ist, dass sie das Armelement an dem ersten Verbinder befestigt, wobei die Befestigungseinheit einen Schaft einschließt, der so konfiguriert ist, dass er die zweite Oberfläche des Armelements kontaktiert, wobei die Befestigungseinheit ein Gewindebolzen ist, wobei eine untere Oberfläche eines Kopfes des Gewindebolzens so konfiguriert ist, dass sie eine obere Oberfläche des Armelements kontaktiert und das Armelement an die erste Oberfläche des ersten Verbinders bindet, oder
wobei der dritte Kontaktabschnitt eine Befestigungseinheit einschließt, die so konfiguriert ist, dass sie das Armelement an dem zweiten Verbinder befestigt, wobei die Befestigungseinheit einen Schaft einschließt, der so konfiguriert ist, dass er die zweite Oberfläche des Armelements kontaktiert, wobei die Befestigungseinheit ein Gewindebolzen ist, wobei eine untere Oberfläche eines Kopfes des Gewindebolzens so konfiguriert ist, dass sie eine obere Oberfläche des Armelements kontaktiert und das Armelement an die erste Oberfläche des zweiten Verbinders bindet.

11. Die Brachytherapie-Applikatorvorrichtung nach einem der vorstehenden Ansprüche, wobei ein distales Ende des ersten Kolpostats und ein distales Ende des zweiten Kolpostats so konfiguriert sind, dass sie durch eine Bewegungsoperation des ersten Verbinders oder des zweiten Verbinders seitlich gespreizt werden, oder
wobei die Applikatorvorrichtung weiter einen ersten eiförmigen Körper umfasst, der mit einem distalen Ende des ersten Kolpostats gekoppelt ist, und einen zweiten eiförmigen Körper, der mit einem distalen Ende des zweiten Kolpostats gekoppelt ist, oder
wobei die Applikatorvorrichtung weiter eine Paddelträgereinheit umfasst, die mit der Verbindungseinheit gekoppelt ist, wobei die Paddelträgereinheit ein Paddel einschließt, das mit einem Paddelschaft gekoppelt ist, oder wobei die Applikatorvorrichtung einen erstes eiförmigen Körper umfasst, der mit einem distalen Ende des ersten Kolpostats gekoppelt ist, und einen zweiten eiförmigen Körper, der mit einem distalen Ende des zweiten Kolpostats gekoppelt ist, und wobei der erste eiförmige Körper und der zweite eiförmige Körper jeweils zylindrisch oder halbzylindrisch sind, oder wobei der erste eiförmige Körper und der zweite eiförmige Körper jeweils kugelförmig oder halbkugelförmig sind oder
wobei die Applikatorvorrichtung weitern ein mit der Verbindungseinheit gekoppeltes Tandem oder ein mit der Verbindungseinheit und dem ersten Verbinder oder dem zweiten Verbinder gekoppeltes Tandem umfasst.

## Revendications

1. - Un dispositif d'applicateur de curiethérapie (1) comprenant :
une source de rayonnement configurée pour être disposée à un point de distribution,
une unité de raccordement incluant un premier raccord (10) couplé à un premier colpostat (11) et un second raccord (13) couplé à un second colpostat (14), dans lequel
le premier raccord (10) inclut une première partie de contact (71) et une partie de corps, la première partie de contact (71) étant fixée de façon immobile à la partie de corps,
le second raccord inclut une deuxième partie de contact (153) et une troisième partie de contact (151), la deuxième partie de contact (153) étant configurée pour venir en contact et venir en prise de façon pivotante avec la première partie de contact (71),
le premier raccord (10) inclut en outre une quatrième partie de contact (41) configurée pour venir en contact avec la troisième partie de contact (151) et
la deuxième partie de contact (153) et la troisième partie de contact (151) sont disposées entre la première partie de contact et la quatrième partie de contact, la première partie de contact (71) étant disposée plus près du point de distribution de la source de rayonnement que les deuxième, troisième ou quatrième parties de contact ne sont disposées par rapport au point de distribution de la source de rayonnement,
**caractérisé en ce que** la première partie de contact (71) pivote autour de la deuxième partie de contact (153) et dans lequel la première partie de contact (71) inclut un élément de douille et la deuxième partie de contact (153) inclut un élément sphérique.

2. - Le dispositif d'applicateur de curiethérapie selon la revendication 1, dans lequel la première partie de contact inclut une partie concave configurée pour venir en prise avec une partie convexe de la deuxième partie de contact.

3. - Le dispositif d'applicateur de curiethérapie selon la revendication 1, dans lequel la deuxième partie de contact est fixée de façon immobile à une partie de corps du second raccord.

4. - Le dispositif d'applicateur de curiethérapie selon la revendication 1, dans lequel la troisième partie de contact inclut un élément de bras, l'élément de bras incluant une première surface configurée pour venir en contact avec une première surface du premier raccord et une seconde surface configurée pour venir en contact avec la quatrième partie de contact ou dans lequel la quatrième partie de contact inclut un élément de bras, l'élément de bras incluant une première surface configurée pour venir en contact avec une première surface du second raccord et une seconde surface configurée pour venir en contact avec la troisième partie de contact.

5. - Le dispositif d'applicateur de curiethérapie selon la revendication 1, dans lequel la troisième partie de contact inclut un élément de bras, l'élément de bras incluant une première surface configurée pour venir en contact avec une première surface du premier raccord et une seconde surface configurée pour venir en contact avec la quatrième partie de contact et
dans lequel la quatrième partie de contact inclut une partie surélevée faisant saillie depuis le premier raccord, la partie surélevée incluant une surface latérale configurée pour venir en prise avec la seconde surface de l'élément de bras ou
dans lequel la quatrième partie de contact inclut une unité de fixation configurée pour fixer l'élément de bras au premier raccord, l'unité de fixation incluant un arbre configuré pour venir en contact avec la seconde surface de l'élément de bras,
dans lequel la première surface de l'élément de bras est presque perpendiculaire à la seconde surface de l'élément de bras et la première surface de l'élément de bras est presque parallèle à la première surface du premier raccord.

6. - Le dispositif d'applicateur de curiethérapie selon la revendication 1, dans lequel la quatrième partie de contact inclut un élément de bras, l'élément de bras incluant une première surface configurée pour venir en contact avec une première surface du second raccord et une seconde surface configurée pour venir en contact avec la troisième partie de contact et
dans lequel la troisième partie de contact inclut une partie surélevée faisant saillie depuis le second raccord, la partie surélevée incluant une surface latérale configurée pour venir en prise avec la seconde surface de l'élément de bras ou
dans lequel la troisième partie de contact inclut une unité de fixation configurée pour fixer l'élément de bras au second raccord, l'unité de fixation incluant un arbre configuré pour venir en contact avec la seconde surface de l'élément de bras, dans lequel la première surface de l'élément de bras est presque perpendiculaire à la seconde surface de l'élément de bras et la première surface de l'élément de bras est presque parallèle à la première surface du second raccord.

7. - Le dispositif d'applicateur de curiethérapie selon la revendication 1, dans lequel la troisième partie de contact inclut un élément de bras, l'élément de bras incluant une première surface configurée pour venir en contact avec une première surface du premier raccord et une seconde surface configurée pour venir en contact avec la quatrième partie de contact et dans lequel la quatrième partie de contact inclut une partie surélevée faisant saillie depuis le premier raccord, la partie surélevée incluant une surface latérale configurée pour venir en prise avec la seconde surface de l'élément de bras, le dispositif d'applicateur comprenant en outre une unité de fixation configurée pour fixer l'élément de bras au premier raccord, l'unité de fixation incluant un arbre agencé sur un côté de l'élément de bras à l'opposé de la partie surélevée, l'élément de bras étant configuré pour passer de manière fonctionnelle entre l'arbre de l'unité de fixation et la surface latérale de la partie surélevée.

8. - Le dispositif d'applicateur de curiethérapie selon la revendication 1, dans lequel la quatrième partie de contact inclut un élément de bras, l'élément de bras incluant une première surface configurée pour venir en contact avec une première surface du second raccord et une seconde surface configurée pour venir en contact avec la troisième partie de contact et dans lequel la troisième partie de contact inclut une partie surélevée faisant saillie depuis le second raccord, la partie surélevée incluant une surface latérale configurée pour venir en prise avec la seconde surface de l'élément de bras,
le dispositif d'applicateur comprenant en outre
une unité de fixation configurée pour fixer l'élément de bras au second raccord, l'unité de fixation incluant un arbre agencé sur un côté de l'élément de bras à l'opposé de la partie surélevée, l'élément de bras étant configuré pour passer de manière fonctionnelle entre l'arbre de l'unité de fixation et la surface latérale de la partie surélevée.

9. - Le dispositif d'applicateur de curiethérapie selon la revendication 7, dans lequel l'unité de fixation est un boulon fileté, une surface inférieure d'une tête du boulon fileté étant configurée pour venir en contact avec une surface supérieure de l'élément de bras et lier l'élément de bras à la première surface du premier raccord ou à la première surface du second raccord.

10. - Le dispositif d'applicateur de curiethérapie selon la revendication 7, dans lequel la quatrième partie de contact inclut une unité de fixation configurée pour fixer l'élément de bras au premier raccord, l'unité de fixation incluant un arbre configuré pour venir en contact avec la seconde surface de l'élément de bras, dans lequel l'unité de fixation est un boulon fileté, une surface inférieure d'une tête du boulon fileté étant configurée pour venir en contact avec une surface supérieure de l'élément de bras et lier l'élément de bras à la première surface du premier raccord ou
dans lequel la troisième partie de contact inclut une unité de fixation configurée pour fixer l'élément de bras au second raccord, l'unité de fixation incluant un arbre configuré pour venir en contact avec la seconde surface de l'élément, dans lequel l'unité de fixation est un boulon fileté, une surface inférieure d'une tête du boulon fileté étant configurée pour venir en contact avec une surface supérieure de l'élément de bras et lier l'élément de bras à la première surface du second raccord.

11. Le dispositif d'applicateur de curiethérapie selon l'une quelconque des revendications précédentes, dans lequel une extrémité distale du premier colpostat et une extrémité distale du second colpostat sont configurées pour être écartées latéralement par une opération de mouvement du premier raccord ou du second raccord ou
dans lequel le dispositif d'applicateur comprend en outre un premier ovoïde couplé à une extrémité distale du premier colpostat et un second ovoïde couplé à une extrémité distale du second colpostat ou
dans lequel le dispositif d'applicateur comprend en outre une unité de support de palette couplée à l'unité de raccordement, l'unité de support de palette incluant une palette couplée à un arbre de palette ou dans lequel le dispositif d'applicateur comprend en outre un premier ovoïde couplé à une extrémité distale du premier colpostat et un second ovoïde couplé à une extrémité distale du second colpostat et dans lequel le premier ovoïde et le second ovoïde sont chacun cylindriques ou semi-cylindriques ou dans lequel le premier ovoïde et le second ovoïde sont chacun sphériques ou semi-sphériques ou
dans lequel le dispositif d'applicateur comprend en outre un tandem couplé à l'unité de raccordement ou un tandem couplé à l'unité de raccordement et au premier raccord ou au second raccord.
